# EUROPEAN PATENT SPECIFICATION

(11) **EP 3 177 225 B1**
(45) Date of publication and mention of the grant of the patent: **22.09.2021**
(21) Application number: 15830479.0
(22) Date of filing: 20.05.2015
(51) Int. Cl.: A61B 17/17, A61B 90/10, A61B 90/11

(54) **STEREOTACTIC TEMPLATE**
STEREOTAKTISCHE SCHABLONE
MODÈLE STÉRÉOTAXIQUE

(30) Priority: 05.08.2014 AU 2014100880; 05.08.2014 AU 2014903034
(43) Date of publication of application: 14.06.2017
(73) Proprietor: D'Urso, Paul S., Richmond, Victoria 3121 (AU)
(72) Inventor: D'Urso, Paul S., Richmond, Victoria 3121 (AU)
(74) Representative: Lincoln IP
(86) International application number: PCT/AU2015/050256
(87) International publication number: WO 2016/019424

(56) References cited:
- WO-A1-00/01316
- WO-A1-2011/029934
- WO-A1-2013/158521
- WO-A2-2012/024281
- CN-A- 103 476 354
- US-A1- 2008 114 370
- US-A1- 2008 171 930
- US-A1- 2012 234 329
- US-A1- 2013 053 854
- US-A1- 2013 274 778
- None

## Description

### FIELD

The present disclosure relates to a stereotactic template for facilitating surgery, particularly minimally invasive spinal fusion surgery.

### BACKGROUND

Minimally Invasive Surgery (MIS) is becoming a standard of care throughout the world. An example of such apparatus is described in international patent publication number WO 2011/029934. To perform minimally invasive percutaneous instrumentation of the spine, cannulated needles, such as Jamshidi needles, are introduced into the pedicles of the relevant vertebrae. Once the Jamshidi needle is in place, a guide wire, such as a K-wire, is introduced through the Jamshidi needle and into the pedicle. The Jamshidi needle is then removed and the guide wire may then be used to guide the placement of a cannulated pedicle screw.

In spinal fusion procedures, the accuracy with which the pedicle screws are inserted in the pedicles of the vertebrae has a direct effect on the surgical outcome. Accurate placement generally involves considerable judgmental skills that have been developed through a lengthy training process. As the impact of misaligning one or more pedicle screws can directly affect patient safety, a number of navigational and trajectory verification approaches have been developed. Some examples of currently available guided screw insertion approaches include intraoperative fluoroscopy, both fluoroscope and computed tomography (CT)-guided computer assisted surgery, electrophysiological monitoring techniques and ultrasonic image-guided pedicle screw insertion.

It would be desirable to provide a device that would assist the surgeon in more accurate placement of screws or other instruments in an anatomical body, such as a vertebral body.

The reference in this specification to any prior publication (or information derived from it), or to any matter which is known, is not, and should not be taken as an acknowledgement or admission or any form of suggestion that that prior publication (or information derived from it) or known matter forms part of the common general knowledge in the field of endeavour to which this specification relates.

### SUMMARY

In one aspect there is provided a stereotactic template for facilitating a surgical procedure, said surgical procedure being spinal surgery, wherein said template comprises at least two hollow trajectory guides sized to receive an instrument for percutaneous introduction into the human body, wherein said template comprises an upper surface and a lower surface, wherein at least part of the lower surface of the template is shaped to match an outer surface of a patient's anatomy such as a skin contour, wherein the template comprises one or more radio-opaque markers characterised in that the trajectory guides are additional radio-opaque markers.

The stereotactic template is patient specific. The stereotactic template may be customized based on one or more parts of a patient's anatomy.

The surgical procedure may be minimally invasive spinal surgery. The surgical procedure may be spinal fusion. The surgical procedure may be transforaminal lumbar interbody fusion (TLIF).

In use, the template may be placed on a patient's skin so that the engineered lower surface is in contact with the patient's skin.

The template may be designed based on patient specific information obtained via, for example, medical imaging, which may enable at least part of the lower surface of the template to be engineered to match a skin contour of the patient, and, further, may enable the at least one trajectory guide to be suitably angled so as to provide a guiding vector from the surface of the patient's skin to a target location on, for example, an anatomical body. The at least one trajectory guide may be suitably angled relative to the lower surface of the template. The at least one trajectory guide may be suitably angled relative to the patient's skin. The anatomical body may be a vertebral body. The vertebral body may be a pedicle.

The at least one trajectory guide may be angled so as to define a vector between an area on the surface of the patient's skin and an anatomical body.

The lower surface of the template and the angle of the at least one trajectory guide may be designed and shaped based on patient specific information. That is, the template may be customized so that at least part of the lower surface of the template matches the contours of a patient's skin and the one or more trajectory guides angled so as to provide a guiding vector from the patient's skin to an anatomical body, such as a vertebral body. The vertebral body may be a pedicle.

The template may comprise at least three, or at least four trajectory guides. The at least three, or at least four trajectory guides may be angled so as to provide guiding vectors from the patient's skin to an anatomical body, such as a vertebral body. The vertebral body may be a pedicle.

Accordingly, the template may facilitate the localisation of skin incisions at an area where the at least one hollow trajectory guide meets the skin and provide trajectory approximation for the placement of instruments, such as guide wires, into an anatomical body, such as a vertebral body, for example a pedicle, by way of, for example, cannulated needles, such as Jamshidi needles. Advantageously, the template improves the accuracy of placement of instruments, such as guide wires, minimising the possibility that further corrective procedures may be required. This reduces operating time and therefore cost and also improves patient safety.

The at least one trajectory guide may be elongate. The at least one trajectory guide may be substantially cylindrical in shape.

The at least one trajectory guide may extend beyond the upper surface of the template. The at least one trajectory guide may extend 1 cm or more, or 2 cm or more beyond the upper surface of the template. The at least one trajectory guide may extend between 0.5 and 5 cm beyond the upper surface of the template.

The internal diameter of the at least one trajectory guide may be between 0.2 and 3 cm or between 0.5 and 2 cm. The internal diameter of the at least one trajectory guide may be greater than 0.2 cm.

The internal dimensions of the at least one trajectory guide may be sized so as to receive an instrument for percutaneous introduction into the human body.

The lower surface of the template which may be engineered to match the contours of a patient's skin may be, at least partly, defined by the ends of the one or more trajectory guides which, in use, face the patient's skin.

The at least one trajectory guide may be angled 45 degrees or less from a vector perpendicular to the plane approximately defined by the surface of the patient's skin.

The at least one trajectory guide may be angled 25 degrees or less from a vector perpendicular to the plane approximately defined by the surface of the patient's skin.

The at least one trajectory guide may be angled 15 degrees or less from a vector perpendicular to the plane approximately defined by the surface of the patient's skin.

The at least one trajectory guide may be angled so that, in use, a vector passing through the hollow trajectory guide extends through the centre of an axis of a pedicle.

The angle of the at least one trajectory guide may be defined by a vector down the centre of the axis of a pedicle and extended to the skin surface and beyond.

The angle may be defined using three-dimensional medical imaging data and computer software.

The fiducial or radio-opaque markers may be used to align the template when contour matching the skin surface to an anatomical body, such as a vertebral body, by the use of image intensification X-rays.

The fiducial markers may be patient specific and incorporated in the device at a pre-operative design stage. Accordingly, the template may comprise one or more fiducial markers specific to a patient's anatomy.

The template may comprise at least two, or at least three, or at least four radio-opaque or fiducial markers.

The fiducial markers may be included in the template to align to the midline of the spine and to the superior endplate of a vertebral body in the design stage, for example by using three-dimensional imaging software. By using X-rays the fiducial markers may be used to accurately align the template with the bony anatomy.

The one or more trajectory guides may be radio-opaque or fiducial markers. The one or more trajectory guides may be used as fiducial markers to align coaxial X-ray images to a pedicle. The X-rays may be obtained in a coaxial plane with the trajectory guides and a "ring" will appear because the centre of a cylindrical trajectory guide is hollow and is surrounded by the template material or barrel which absorbs the X-rays. Accordingly, the ring may be aligned with the pedicle, which itself is also a ring comprising cortical hard bone surrounding less dense cancellous bone. Therefore the trajectory guide ring may superimpose itself with the pedical ring when they are accurately aligned and the X-rays are obtained in a coaxial plane to both the template and the pedicle.

The inclusion of fiducial or radio-opaque markers in the template enable the template to be accurately positioned on a patient's skin. The markers are patient specific and are designed into the template based on patient medical imaging. Proper positioning of the template on the patient's skin is advantageous as it improves the accuracy of placement of instruments, minimising the possibility that further corrective procedures may be required. This reduces operating time and therefore cost and also improves patient safety.

The template may comprise one or more fiducial or radio-opaque markers on the surface of the template and additionally the one or more trajectory guides may be fiducial or radio-opaque markers.

The template may comprise any combination of the hereinbefore disclosed embodiments.

In another aspect there is provided a method for designing a stereotactic template comprising the steps of:
a) determining two or more trajectories from a relevant anatomical body to a patient's skin surface using preoperative medical imaging;
b) determining the contours of a patient's skin at an area where the template will be utilized using preoperative medical imaging; and
c) designing a template comprising at least two hollow trajectory guide, sized to receive an instrument for percutaneous introduction into the human body, wherein said template has an upper surface and a lower surface and wherein the lower surface of the template matches a skin contour of a patient
   wherein the template comprises one or more radio-opaque markers characterised in that
   one or more of the trajectory guides are additional radio-opaque markers.

The at least two trajectory guide may be angled so as to define a vector between an area on the surface of the patient's skin and a relevant anatomical body.

The method may also comprise the step of including one or more radio-opaque or fiducial markers in the template using preoperative medical imaging.

The method may also comprise the step of including one or more radio-opaque or fiducial markers in the one or more trajectory guides using preoperative medical imaging

In another aspect there is provided a method for manufacturing a stereotactic template comprising the steps of:
a) determining two or more trajectories from a relevant anatomical body to the patient's skin surface using preoperative medical imaging;
b) determining the contours of a patient's skin at an area where the template will be utilized using preoperative medical imaging;
c) designing a template comprising at least two hollow trajectory guide, sized to receive an instrument for percutaneous introduction into the human body, wherein said template has an upper surface and a lower surface wherein the lower surface of the template matches a skin contour of a patient and;
d) manufacturing the template
   wherein the template comprises one or more radio-opaque markers
   characterised in that
   one or more of the trajectory guides are additional radio-opaque markers.

The at least two trajectory guide may be angled so as to define a vector between an area on the surface of the patient's skin and a relevant anatomical body.

In any of the hereinbefore disclosed embodiments the anatomical body may be a vertebral body. The vertebral body may be a pedicle.

In any of the hereinbefore disclosed embodiments the medical imaging may be computed tomography.

In any of the hereinbefore disclosed embodiments the template may be manufactured using rapid prototype technology.

In any of the hereinbefore disclosed embodiments the template may be manufactured using three-dimensional printing.

In any of the hereinbefore disclosed embodiments the trajectories may be represented as three-dimensional images with the aid of suitable computer software. The trajectories may be determined to meet the skin at particular entry points.

The template may be manufactured from a suitable compatible polymer, such as, for example polyether ether ketone.

In another aspect there is provided a use of a stereotactic template as hereinbefore disclosed in a surgical procedure. The surgical procedure may be spinal surgery. The surgical procedure may be minimally invasive spinal surgery. The surgical procedure may be spinal fusion. The surgical procedure may be transforaminal lumbar interbody fusion (TLIF).

In another aspect there is provided a method of using a stereotactic template in a surgical procedure comprising the steps of:
a) providing a stereotactic template according to any one of the hereinbefore disclosed embodiments; and
b) positioning said template on a patient's skin so that the lower surface of the template aligns with a skin contour of the patient and at least one trajectory guide facilitates percutaneous introduction of an instrument in an anatomical body.

The surgical procedure may be spinal surgery. The surgical procedure may be minimally invasive spinal surgery. The surgical procedure may be spinal fusion. The surgical procedure may be transforaminal lumbar interbody fusion (TLIF).

The anatomical body may be a vertebral body. The anatomical body may be a pedicle.

The method may further comprise the step of aligning the at least one trajectory guide via a vector passing through the trajectory guide to the anatomical body.

Throughout this specification, use of the terms "comprises" or "comprising" or grammatical variations thereon shall be taken to specify the presence of stated features, integers, steps or components but does not preclude the presence or addition of one or more other features, integers, steps, components or groups thereof not specifically mentioned.

### BRIEF DESCRIPTION OF THE DRAWINGS

Figure 1 (a) illustrates a plan view of a template according to an embodiment of the present disclosure.
Figure 1 (b) illustrates a plan view of a template according to an embodiment of the present disclosure.
Figure 1(c) illustrates a side elevation of a template according to an embodiment of the present disclosure.
Figure 1(d) illustrates a side elevation of a template according to an embodiment of the present disclosure.
Figure 2 illustrates a computer simulation of a template according to an embodiment of the present disclosure.
Figure 3 illustrates a computer simulation of a template according to an embodiment of the present disclosure.
Figure 4 illustrates a computer simulation of a template according to alternative embodiment of the present disclosure.
Figure 5 illustrates a computer simulation of a template according to alternative embodiment of the present disclosure.
Figure 6 is an X-ray image of a template in position and showing radio-opaque markers.

### DESCRIPTION OF EXEMPLARY EMBODIMENTS

Before the present devices and/or methods are disclosed and described, it is to be understood that unless otherwise indicated this invention is not limited to specific devices, components, designs, methods, or the like, as such may vary, unless otherwise specified. It is also to be understood that the terminology used herein is for the purpose of describing particular embodiments only and is not intended to be limiting.

It must also be noted that, as used in the specification and the appended claims, the singular forms 'a', 'an' and 'the' include plural referents unless otherwise specified. Thus, for example, reference to 'a guide wire' may include more than one guide wires, and the like.

Disclosed herein are advantageous devices and methods for performing surgery, particularly spinal fusion surgery, wherein the methods do not form part of the invention as claimed. The devices and methods are based on patient specific information or patient specific anatomy. Accordingly, a patient specific template customized to a patient's anatomy is provided which reduces operating time and improves patient safety.

In an exemplary embodiment, from patient specific preoperative computed tomography scan information, trajectories to the relevant pedicles of a patient's spine may be determined and such trajectories then reproduced as three-dimensional images using suitable computer software. Such trajectories may be determined to meet the skin at particular entry points. A template may then be contour matched to the surface of the patient's skin to incorporate such entry points on the skin for guide wires as well as by virtue of one or more small hollow tubes or trajectory guides extending above the level of the template. The template is designed with computer aided design software based on the trajectories determined from the patient's preoperative imaging.

The template may be subsequently manufactured using rapid prototype technology and printed in three-dimensions. Radio-opaque markers may be included in the template to mark the midline as well as the level of spinal surgery according to the end plate of the vertebral body. During the manufacturing process, radio-opaque markers may be incorporated into the template. These markers may be patient and procedure specific. At the time of surgery the template is placed on the patient's skin after the patient has been placed into position for the procedure. The template may then be used to facilitate markings on the skin for entry points for the placement of guide-wires through the skin. The template may also incorporate trajectory guides extending above the level of the template so as to facilitate placement of guide wires, such as K-wires, via Jamshidi needles. The template is positioned using intraoperative image intensification to localise the template along the axis of the spine in the midline as well as at the desired level of the spine according to the end plate of the selected vertebra and by alignment of the radio-opaque markers.

The template may be used to mark trajectories and entry points of vectors to place pedicle screws into the lumbar spine. The template may be designed with a combination of computer aided design software and preoperative medical imaging data capturing anatomical morphology. Radio-opaque markers may be incorporated in, for example, channels in the template which may orientate the template to the patient's midline and to a specific level of the spine when image intensification or X-ray radiation is used to image and align both the template and the patient's spine simultaneously in a predetermined way.

Computer aided design (CAD) software may be used to construct a template to surface match the contour of the skin of the patient and integrate a cylindrical vector extension approximately 2 cm beyond the surface of the skin which captures the trajectory of the vector in a cylindrical barrel which is attached to the contour matched template. Positions of radio-opaque alignment markers may be incorporated into the design.

The CAD data may be exported to a three-dimensional printing machine so that the template may be manufactured.

The template may be contoured matched to the patient's skin and aligned to the patient's spinal anatomy using radiant energy, such as image intensification, so that the radio-opaque markers are aligned with the image of the patient's spine in a predetermined way.

The template trajectory guides may be used to assist with placement of Jamshidi needles into the pedicles of the spine, with the assistance of image intensification if necessary.

Once the pedicle is located, a K-wire may be introduced through the Jamshidi needle into the cancellous bone of the vertebral body to replicate the vector into the pedicle from the skin surface. Once the K-wire is introduced the template may be removed.

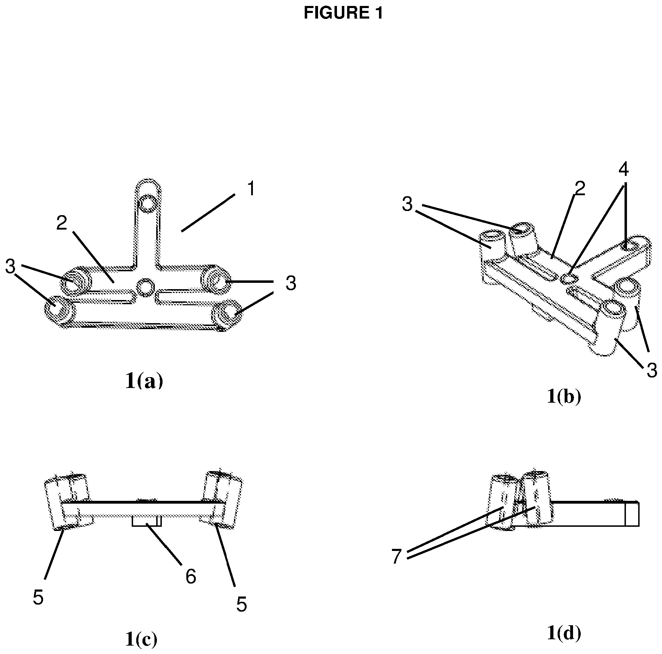

Referring to Figure 1(a), template (1) has an upper surface (2) and four trajectory guides (3) which are cylindrical in shape and hollow so as to define a channel through the upper surface to the lower surface which, in use, face a patient's skin. In this embodiment the trajectory guides extend above the upper surface (2).

Referring to Figure 1(b), the trajectory guides (3) extend above the upper surface (2) of the template. Fiducial markers are located at (4).

Referring to Figure 1(c), the bottom faces of the trajectory guides (5) are collectively engineered so as to define the lower surface of the template which, in use, is in contact with patient's skin. Support part (6) is also engineered to match the contours of a patient's skin and in this embodiment forms part of the lower surface. It can be seen that the trajectory guides are suitably angled so as to define a predetermined pathway from the surface of the patient's skin to a relevant vertebral body.

Referring to Figure 1(d), the dotted lines (7) through the trajectory guides represent vectors running from the surface of the skin to a relevant vertebral body.

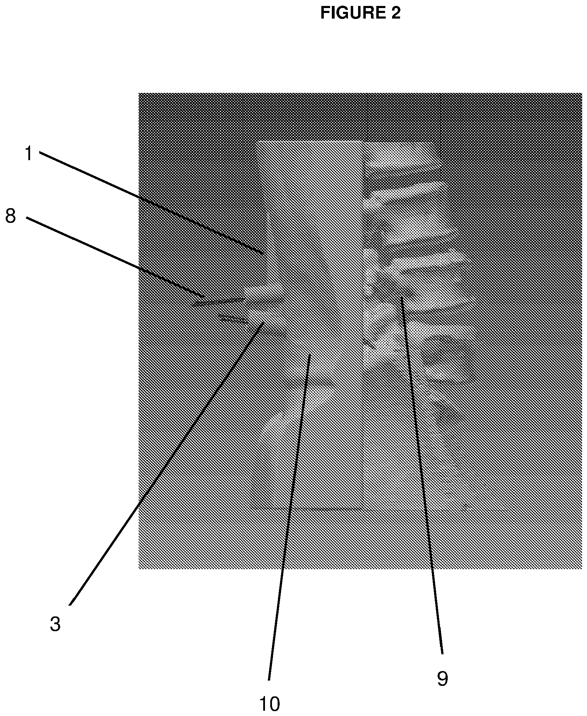

Referring to Figure 2, template (1) having trajectory guides (3) is modelled in use on the surface of a patient's skin (10). The spinal structure is shown as (9). The angled nature of the trajectory guides is clearly visible. Wires (8) are in place within the hollow trajectory guides.

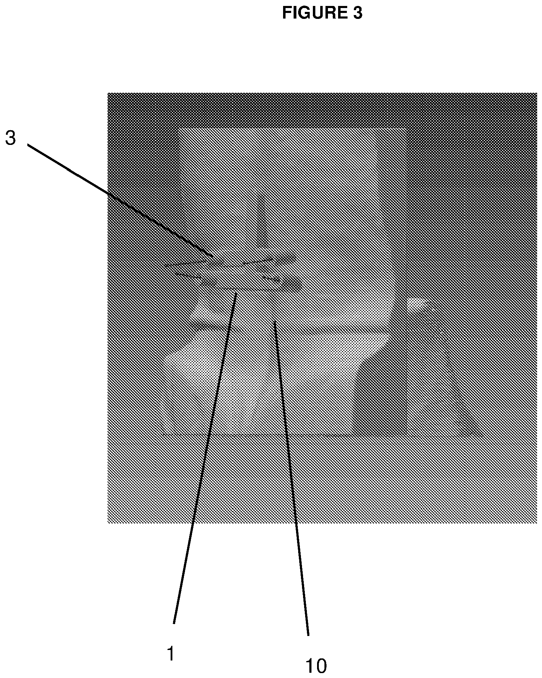

Referring to Figure 3, a further view of a modelled skin surface (10) having a template (1) in position is provided. Again, the angled nature of the trajectory guides (3) is clearly visible.

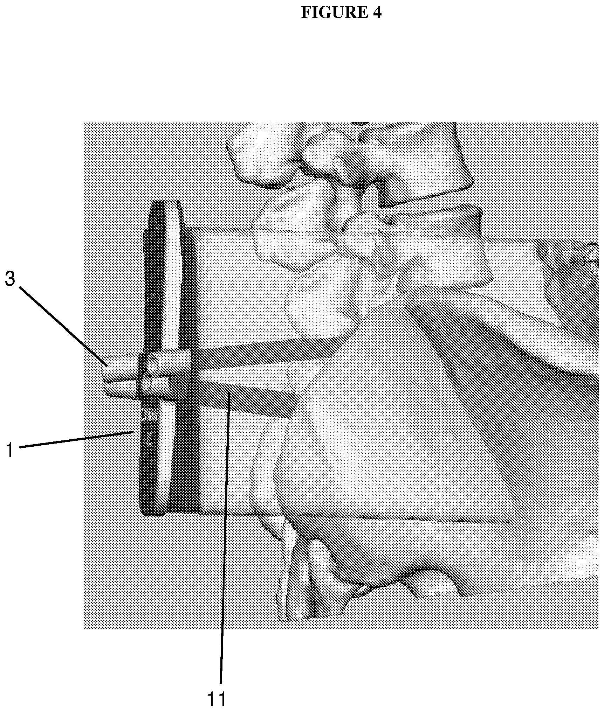

Figure 4 illustrates another embodiment of a template (1) positioned on a skin surface. The tissue surrounding the spinal anatomy is cutaway so as to reveal the vertebral bodies. Trajectory guides (3) project from the upper surface of the template. The solid lines (11) illustrate alignment of the vectors running through the trajectory guides with the target vertebral body.

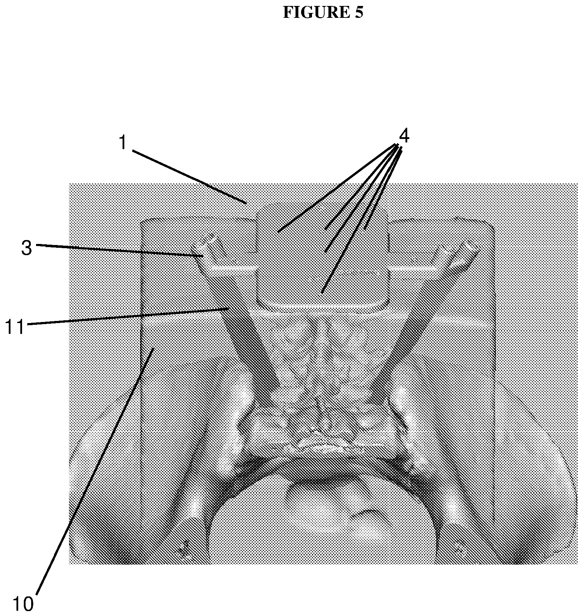

Figure 5 is a plan view of the skin template positioned on a skin surface (10). Five fiducial markers (4) are present.

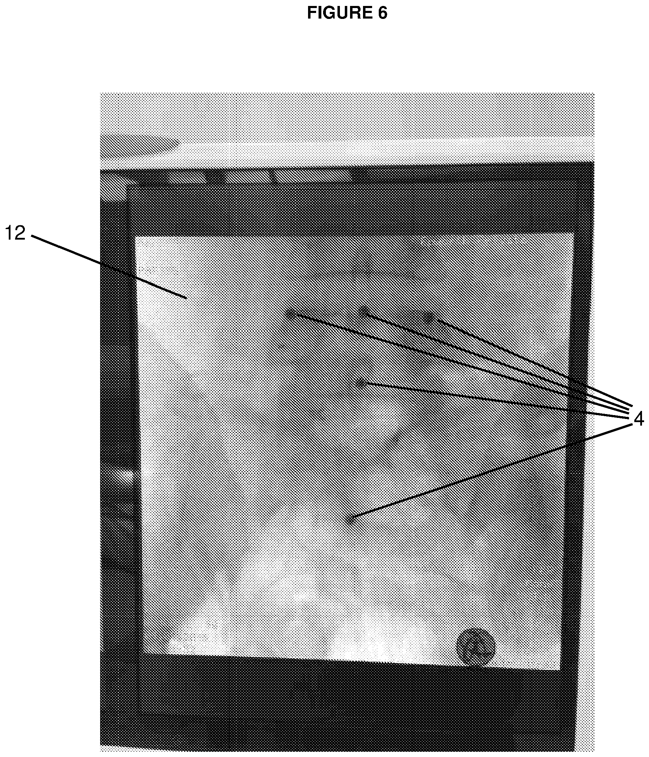

Figure 6 is an X-ray image (12) of a template showing five fiducial markers (4). The template is positioned on a patient's outer anatomy.

While the foregoing description has focused on spinal surgery, it is contemplated that the templates and methods described herein may find use in a wide range of surgical applications, where localization of anatomical bodies is required. Thus, where it is desired to access a surgical target site via an instrument, the template of the present disclosure may be used.

It is to be understood that while the present disclosure has been described in conjunction with the specific embodiments thereof, the foregoing description is intended to illustrate and not limit the scope of the disclosure. Other aspects, advantages and modifications will be apparent to those skilled in the art to which the disclosure pertains. Therefore, the above examples are put forth so as to provide those skilled in the art with a complete disclosure and description of how to make and use the disclosed devices, and are not intended to limit the scope of the disclosure.

For the sake of brevity, only certain ranges are explicitly disclosed herein. However, ranges from any lower limit may be combined with any upper limit to recite a range not explicitly recited, as well as, ranges from any lower limit may be combined with any other lower limit to recite a range not explicitly recited, in the same way, ranges from any upper limit may be combined with any other upper limit to recite a range not explicitly recited.

## Claims

1. A stereotactic template (1) for facilitating a surgical procedure, said surgical procedure being spinal surgery, wherein said template (1) comprises at least two hollow trajectory guides (3) sized to receive an instrument for percutaneous introduction into the human body,
wherein said template (1) comprises an upper surface (2) and a lower surface (5) wherein at least part of the lower surface (5) of the template (1) is shaped to match an outer surface of a patient's anatomy, namely the skin contour,
wherein the template (1) comprises one or more radio-opaque markers (4) **characterised in that**
the trajectory guides (3) are additional radio-opaque markers.

2. A template (1) according to claim 1, wherein, in use, the template (1) is placed on a patient's skin so that the shaped lower surface (5) is in contact with the patient's skin.

3. A template (1) according to claim 1 or claim 2, wherein the template (1) is designed based on patient specific information obtained via medical imaging.

4. A template (1) according to any one of claims 1 to 3, wherein the surgical procedure is spinal fusion surgery, preferably minimally invasive spinal fusion surgery.

5. A template (1) according to any one of claims 1 to 4 wherein the at least two trajectory guides (3) are suitably angled so as to provide a guiding vector from the surface of the patient's skin to a target location on an anatomical body, preferably a vertebral body.

6. A template (1) according to any one of claims 1 to 5 wherein the at least one trajectory guides (3) are suitably angled relative to the lower surface (5) of the template (1) or is suitably angled relative to the patient's skin.

7. A template (1) according to any one of claims 1 to 6 wherein the at least two trajectory guides (3) are angled so as to define a vector between an area on the surface of the patient's skin and a vertebral body.

8. A template (1) according to any one of claims 1 to 7 comprising at least three, or at least four trajectory guides (3) and wherein the at least three, or at least four trajectory guides (3) preferably are angled so as to provide guiding vectors from the patient's skin to a vertebral body.

9. A template (1) according to any one of claims 1 to 8 wherein the lower surface (5) of the template (1), which is engineered to match the contours of a patient's skin is, at least partly, defined by the ends of the two or more trajectory guides (3) which, in use, face the patient's skin.

10. A template (1) according to any one of claims 1 to 9 wherein the at least two trajectory guides (3) are angled so that, in use, vectors passing through the hollow trajectory guides (3) extend through the centre of an axis of a vertebral body.

11. A template (1) according to any one of claims 1 to 10 wherein the radio-opaque markers (4) are configured to be used to align the template (1) when contour matching the skin surface to a vertebral body by the use of image intensification X-rays.

12. A method for designing a stereotactic template (1) comprising the steps of:
a) determining two or more trajectories from a relevant anatomical body to a patient's skin surface using preoperative medical imaging;
b) determining the contours of a patient's skin at an area where the template (1) will be utilized using preoperative medical imaging; and
c) designing a template (1) comprising at least two hollow trajectory guides (3), sized to receive an instrument for percutaneous introduction into the human body, wherein said template (1) has an upper surface (2) and a lower surface (5) and wherein at least part of the lower surface (5) of the template (1) matches a skin contour of a patient and
wherein the template (1) comprises one or more radio-opaque markers (4)
**characterised in that**
the trajectory guides (3) are additional radio-opaque markers.

13. A method for manufacturing a stereotactic template (1) comprising the steps of:
a) determining two or more trajectories from a relevant anatomical body to the patient's skin surface using preoperative medical imaging;
b) determining the contours of a patient's skin at an area where the template (1) will be utilized using preoperative medical imaging;
c) designing a template comprising at least two hollow trajectory guides (3), sized to receive an instrument for percutaneous introduction into the human body, wherein said template (1) has an upper surface (2) and a lower surface (5), and wherein at least part of the lower surface (5) of the template matches a skin contour of a patient and;
d) manufacturing the template (1)
wherein the template (1) comprises one or more radio-opaque markers (4)
**characterised in that**
the trajectory guides (3) are additional radio-opaque markers.

## Patentansprüche

1. Eine stereotaktische Schablone (1) zur Erleichterung eines chirurgischen Verfahrens, wobei das chirurgische Verfahren ein wirbelsäulenchirurgischer Eingriff ist, wobei die Schablone (1) mindestens zwei hohle Trajektorienführungen (3) umfasst, die so bemessen sind, dass sie ein Instrument zur perkutanen Einführung in den menschlichen Körper aufnehmen können,
wobei die Schablone (1) eine obere Fläche (2) und eine untere Fläche (5) umfasst. wobei zumindest ein Teil der unteren Oberfläche (5) der Schablone (1) so geformt ist, dass sie einer äußeren Oberfläche der Anatomie eines Patienten, nämlich der Hautkontur, entspricht,
wobei die Schablone (1) einen oder mehrere röntgendichte Marker (4) umfasst, **dadurch gekennzeichnet, dass**
die Trajektorienführungen (3) zusätzliche röntgendichte Marker sind.

2. Eine Schablone (1) nach Anspruch 1, wobei die Schablone (1) im Gebrauch so auf der Haut eines Patienten platziert wird, dass die geformte untere Fläche (5) in Kontakt mit der Haut des Patienten ist.

3. Eine Schablone (1) nach Anspruch 1 oder Anspruch 2, wobei die Schablone (1) basierend auf patientenspezifischen Informationen entworfen ist, die über medizinische Bildgebung erhalten wurden.

4. Eine Schablone (1) nach einem der Ansprüche 1 bis 3, wobei das chirurgische Verfahren eine Wirbelsäulenfusionschirurgie ist, vorzugsweise eine minimal invasive Wirbelsäulenfusionschirurgie.

5. Eine Schablone (1) nach einem der Ansprüche 1 bis 4, wobei die mindestens zwei Trajektorienführungen (3) geeignet abgewinkelt sind, um einen Führungsvektor von der Oberfläche der Haut des Patienten zu einem Zielort an einem anatomischen Körper, vorzugsweise einem Wirbelkörper, bereitzustellen.

6. Eine Schablone (1) nach einem der Ansprüche 1 bis 5, wobei die mindestens eine Trajektorienführung (3) in geeigneter Weise relativ zur unteren Fläche (5) der Schablone (1) oder in geeigneter Weise in Bezug auf die Haut des Patienten abgewinkelt ist.

7. Eine Schablone (1) nach einem der Ansprüche 1 bis 6, wobei die mindestens zwei Trajektorienführungen (3) so abgewinkelt sind, dass sie einen Vektor zwischen einem Bereich auf der Hautoberfläche des Patienten und einem Wirbelkörper definieren.

8. Eine Schablone (1) nach einem der Ansprüche 1 bis 7, die mindestens drei oder mindestens vier Trajektorienführungen (3) umfasst, und wobei die mindestens drei oder mindestens vier Trajektorienführungen (3) vorzugsweise so abgewinkelt sind, dass sie Führungsvektoren von der Haut des Patienten zu einem Wirbelkörper bereitstellen.

9. Eine Schablone (1) nach einem der Ansprüche 1 bis 8, wobei die untere Fläche (5) der Schablone (1), die so konstruiert ist, dass sie den Konturen der Haut eines Patienten entspricht, zumindest teilweise durch die Enden der zwei oder mehr Trajektorienführungen (3), die im Gebrauch der Haut des Patienten zugewandt sind, definiert ist.

10. Eine Schablone (1) nach einem der Ansprüche 1 bis 9, wobei die mindestens zwei Trajektorienführungen (3) so abgewinkelt sind, dass sich im Gebrauch durch die hohlen Trajektorienführungen (3) hindurchtretende Vektoren durch die Mitte einer Achse eines Wirbelkörpers erstrecken.

11. Eine Schablone (1) nach einem der Ansprüche 1 bis 10, wobei die röntgendichten Marker (4) so konfiguriert sind, dass sie verwendet werden können, um die Schablone (1) auszurichten, wenn die Kontur der Hautoberfläche unter Verwendung von Bildintensivierungs-Röntgenbildern an einen Wirbelkörper angepasst wird.

12. Ein Verfahren zum Entwurf einer stereotaktischen Vorlage (1), das die folgenden Schritte umfasst:
a) Bestimmung von zwei oder mehr Trajektorien von einem relevanten anatomischen Körper zur Hautoberfläche eines Patienten unter Verwendung einer präoperativen medizinischen Bildgebung;
b) Bestimmung der Konturen der Haut eines Patienten in einem Bereich, in dem die Schablone (1) verwendet wird, unter Verwendung einer präoperativen medizinischen Bildgebung und
c) Entwurf einer Schablone (1), die mindestens zwei hohle Trajektorienführungen (3) umfasst, die so bemessen sind, dass sie ein Instrument zur perkutanen Einführung in den menschlichen Körper aufnehmen können, wobei die Schablone (1) eine obere Fläche (2) und eine untere Fläche (5) aufweist, und wobei mindestens ein Teil der unteren Fläche (5) der Schablone (1) einer Hautkontur eines Patienten entspricht, und
wobei die Schablone (1) einen oder mehrere röntgendichte Marker (4) umfasst,
**dadurch gekennzeichnet, dass**
die Trajektorienführungen (3) zusätzliche röntgendichte Marker sind.

13. Ein Verfahren zur Herstellung einer stereotaktischen Schablone (1), das die folgenden Schritte umfasst:
a) Bestimmung von zwei oder mehr Trajektorien von einem relevanten anatomischen Körper zur Hautoberfläche des Patienten unter Verwendung einer präoperativen medizinischen Bildgebung;
b) Bestimmung der Konturen der Haut eines Patienten in einem Bereich, in dem die Schablone (1) verwendet wird, unter Verwendung einer präoperativen medizinischen Bildgebung;
c) Entwurf einer Schablone, die mindestens zwei hohle Trajektorienführungen (3) umfasst, die so bemessen sind, dass sie ein Instrument zur perkutanen Einführung in den menschlichen Körper aufnehmen können, wobei die Schablone (1) eine obere Fläche (2) und eine untere Fläche (5) aufweist, und wobei mindestens ein Teil der unteren Fläche (5) der Schablone einer Hautkontur eines Patienten entspricht, und
d) Herstellung der Schablone (1),
wobei die Schablone (1) einen oder mehrere röntgendichte Marker (4) umfasst,
**dadurch gekennzeichnet, dass**
die Trajektorienführungen (3) zusätzliche röntgendichte Marker sind.

## Revendications

1. Un modèle stéréotaxique (1) permettant de faciliter une procédure chirurgicale, ladite procédure chirurgicale étant une chirurgie spinale, où ledit modèle (1) comprend au moins deux guides de trajectoire creux (3) dimensionnés pour recevoir un instrument permettant l'introduction percutanée dans le corps humain,
où ledit modèle (1) comprend une surface supérieure (2) et une surface inférieure (5) où au moins une partie de la surface inférieure (5) du modèle (1) est façonnée pour correspondre à la surface extérieure de l'anatomie du patient, à savoir le contour de la peau,
où le modèle (1) comprend un ou plusieurs marqueurs radio-opaques (4), **caractérisé par le fait que**
les guides de trajectoire (3) sont des marqueurs radio-opaques supplémentaires.

2. Le modèle (1) de la revendication 1, où, dans le cadre de l'utilisation, le modèle (1) est placé sur la peau du patient de sorte que la surface inférieure façonnée (5) soit en contact avec la peau du patient.

3. Le modèle (1) de la revendication 1 ou 2, où le modèle (1) est conçu sur la base d'informations spécifiques au patient obtenues par imagerie médicale.

4. Le modèle (1) de l'une des revendications 1 à 3, où la procédure chirurgicale est une chirurgie de fusion spinale, de préférence une chirurgie de fusion spinale miniinvasive.

5. Le modèle (1) de l'une des revendications 1 à 4, où au moins deux guides de trajectoire (3) sont inclinés de manière à fournir un vecteur de guidage depuis la surface de la peau du patient jusqu'à un emplacement cible sur un corps anatomique, de préférence un corps vertébral.

6. Le modèle (1) de l'une des revendications 1 à 5, où au moins un guide de trajectoire (3) est convenablement incliné par rapport à la surface inférieure (5) du modèle (1) ou est convenablement incliné par rapport à la peau du patient.

7. Le modèle (1) de l'une des revendications 1 à 6, où au moins deux guides de trajectoire (3) sont inclinés de manière à définir un vecteur entre une zone de la surface de la peau du patient et un corps vertébral.

8. Le modèle (1) de l'une des revendications 1 à 7 comprenant au moins trois ou quatre guides de trajectoire (3), où les trois ou quatre guides de trajectoire (3) sont de préférence inclinés de manière à fournir des vecteurs de guidage depuis la peau du patient jusqu'à un corps vertébral.

9. Le modèle (1) de l'une des revendications 1 à 8, où la surface inférieure (5) du modèle (1), qui est conçue pour s'adapter aux contours de la peau du patient, est au moins partiellement définie par les extrémités de deux ou plusieurs guides de trajectoire (3) qui, dans le cadre de l'utilisation, font face à la peau du patient.

10. Le modèle (1) de l'une des revendications 1 à 9, où au moins deux guides de trajectoire (3) sont inclinés de sorte que, dans le cadre de l'utilisation, les vecteurs passant par les guides de trajectoire creux (3) s'étendent à travers le centre d'un axe d'un corps vertébral.

11. Le modèle (1) de l'une des revendications 1 à 10, où les marqueurs radio-opaques (4) sont configurés pour être utilisés afin d'aligner le modèle (1) lors de l'adaptation de la surface de la peau à un corps vertébral au moyen de rayons X à intensification d'image.

12. Un procédé de conception d'un modèle stéréotaxique (1) comprenant les étapes suivantes :
a) déterminer deux trajectoires ou plus depuis un corps anatomique pertinent jusqu'à la surface de la peau du patient au moyen de l'imagerie médicale préopératoire ;
b) déterminer les contours de la peau du patient dans une zone où sera utilisé le modèle (1) au moyen de l'imagerie médicale préopératoire ; et
c) concevoir un modèle (1) comprenant au moins deux guides de trajectoire creux (3) dimensionnés pour recevoir un instrument permettant l'introduction percutanée dans le corps humain, où ledit modèle (1) présente une surface supérieure (2) et une surface inférieure (5) et où au moins une partie de la surface inférieure (5) du modèle (1) correspond au contour de la peau du patient ; et
où le modèle (1) comprend un ou plusieurs marqueurs radio-opaques (4) **caractérisé par le fait que**
les guides de trajectoire (3) sont des marqueurs radio-opaques supplémentaires.

13. Un procédé de fabrication d'un modèle stéréotaxique (1) comprenant les étapes suivantes :
a) déterminer deux trajectoires ou plus depuis un corps anatomique pertinent jusqu'à la surface de la peau du patient au moyen de l'imagerie médicale préopératoire ;
b) déterminer les contours de la peau du patient dans une zone où sera utilisé le modèle (1) au moyen de l'imagerie médicale préopératoire ;
c) concevoir un modèle comprenant au moins deux guides de trajectoire creux (3) dimensionnés pour recevoir un instrument permettant l'introduction percutanée dans le corps humain, où ledit modèle (1) présente une surface supérieure (2) et une surface inférieure (5) et où au moins une partie de la surface inférieure (5) du modèle correspond au contour de la peau du patient ; et
d) fabriquer le modèle (1)
où le modèle (1) comprend un ou plusieurs marqueurs radio-opaques (4) **caractérisé par le fait que**
les guides de trajectoire (3) sont des marqueurs radio-opaques supplémentaires.
